# EUROPEAN PATENT APPLICATION

(11) **EP 1 249 163 A1**
(43) Date of publication of application: **16.10.2002**
(21) Application number: 01108825.9
(22) Date of filing: 09.04.2001
(51) Int. Cl.: A01M 1/20, A61L 9/03

(54) **Thermal vaporizer for a liquid formulation comprising a volatile active**

(71) Applicant: Zelnova, S.A., 36400 Porrino (Pontevedra) (ES)
(72) Inventor: Brown, Colin William, Egham, Surrey TW20 8LP (GB); Hart, Gerald Leslie, Surbiton, Surrey KT5 8BD (GB); Naish, Guy, Narborough, Leicestershire LE9 5DD (GB)
(74) Representative: Davila Baz, Angel

(57) **Abstract**

The present invention is related to a thermal vaporizer (A) for a liquid formulation comprising a volatile active which can to run at two different temperatures. This is achieved by an activating means (20) for two different predetermined heating temperatures of a heater unit (2), said activating means (20) being actuated by a container (50).

## Description

The present invention is related to a thermal vaporizer for a liquid formulation comprising a volatile active which can be operated at two different temperatures.

The use of thermal vaporizers to dispense volatile actives is well known. Such thermal vaporizer known up-to date in the prior art comprise a wick inserted into a container with a reservoir of liquid volatile active, a heater unit and as electric pins which are either fixed, as described in US 4,467,177 or can be rotated such that the wick can be orientated perpendicular to the ground as described in US 5,647,053, US 5,290,546, US 5,038,394, US 5,095,647 and in EP-A 0 962 132. In all cases, the heater unit of the thermal vaporizer runs at one fixed predetermined temperature.

There are thermal vaporizers available which are designed to control the evaporation intensity by moving either the container and wick in relation to the heater unit, as described in EP-A 0 962 132 or by moving the heater unit in relation to the wick, as is the case with a commercially available air freshener (Ambi-Pur by Sara Lee). In these cases, the evaporation intensity is controlled by exposing a greater or smaller surface of the wick to the heater unit but the temperature of the unit is still fixed.

Thermal vaporizer for dispensing an insecticide often use ceramic or graphite-based wicks and run at temperatures typically in the region of 110 to 140°C. Fragrance dispensing thermal vaporizer run generally at lower temperatures, utilizing cellulose or asbestos wicks. As a result of the different wicks and operating temperatures, different heater units have customarily been designed for each end use.

It is an object of the present invention to provide a thermal vaporizer which can operate at two different temperatures, and therefore being able to evaporate different volatile actives.

It is further an object of the present invention to provide a thermal vaporizer which runs at the right temperature.

The problems have been resolved by a thermal vaporizer for a liquid formulation, comprising a volatile active, with a housing having a heater unit for evaporating the liquid formulation, a mounting for a container for the liquid formulation to be disposed under said heater unit, a passage for a wick to be heated by the heater unit, at least one outlet for the evaporated volatile active, an electrical contact connected to said heater unit, characterized by activating means for two different predetermined heating temperatures of the heater unit said activating means being actuated by the container.

The thermal vaporizer according to the present invention can therefore be used for the dispensing of different volatile actives such as insecticides and fragrances. Said thermal vaporizer is safe and there is no danger that the heater unit could run at the incorrect temperature for a given volatile active.

The activating means which actuate the heater unit at one predetermined temperature when the container is fixed at any position of the mounting of the thermal vaporizer. The activating means is preferably a internal switch which is mechanically actuated, e.g. by pressing or by throwing while fixing the container. The activation of the activating means is achieved for example by having an additional molding on the container which engages with the switch or an additional collar that can be placed over the neck of the container. A container without molding or additional neck collar causes the unit to run at the other temperature after having fixed the container into the mounting. In another preferred embodiment of the present invention at least two circuits are present and said circuits are interrupted. At least one of said interrupted circuits is closed by a metal connector on the container when the container is fixed into the mounting.

The wick to be provided on the container may be made of various materials commonly used. Ceramic or graphite-based wicks are preferred.

The heater to be used in the thermal vaporizer is an electric heater which produces heat when connected to a voltage source. In a preferred embodiment the heater unit has a annular shape in which the wick can be inserted concentrically.

During operation of the apparatus, the volatile active in the container is drawn into the wick by capillarity, starting from an area of the wick near the base of the container, where the temperature is lower until it reaches an area adjacent to the heater unit at a distance from said base and outside said container where the temperature is higher.

The volatile actives of the thermally vaporizable liquid formulation are preferable selected from the group of fragrances, insecticides, bactericides, repellents, drugs, herbicides, and fungicides. These volatile actives may be carried by a volatile solvent, for example water, ethanol, isoparaffin or glycol ethers.

The container for the thermal vaporizer according to the present invention comprises means which actuate the activating means of the heater unit when the container is fixed into the mounting. In a preferred embodiment the means is a molding or an electrical contact. In an preferred embodiment the container is a conventional bottle or a similar device sealed with closure which holds the wick firmly in place. Preferably the closure is not easily removable and cooperates with the wick in such a way that the wick is not easily removable from the container. The container comprises further a part which cooperates in a known fashion with the mounting of the thermal vaporizer. In a preferred embodiment the container is fixed into the mounting of the thermal vaporizer by screw-thread engagement between an internally threaded portion of the mounting of the thermal vaporizer and an externally threaded portion of the container. Since the container is fixed directly into the mounting of the thermal vaporizer, the container is easy to fit in and to remove. An alternative to a screw-thread engagement is to have a snap fitting such that the container is pushed, not screwed into place.

The thermal vaporizer according to the present invention has preferably has a starting means on the mounting which is actuated when a container is fixed into the mounting so that the heater unit of the thermal vaporizer can only work when a container is fixed.

In another preferred embodiment the thermal vaporizer according to the present invention has an switch mounted on the housing for turning on an off the heater unit of the thermal vaporizer.

Various embodiments of the invention will be described below with reference to the drawings.

Fig. 1 shows a cross-sectional view of the thermal vaporizer.

Fig. 2 shows a cross-sectional view of a special embodiment of the mounting.

Fig. 3 shows a cross-sectional view of a special embodiment of the means of the container.

Fig. 4a & 4b show cross-sectional views of the container within the mounting.

Fig. 5 shows a cross-sectional view of another preferred embodiment of the mounting.

Fig. 6 shows a cross-sectional view of another preferred embodiment of the means of the container.

Fig. 7a & 7b show cross-sectional views of the container within the mounting.

Fig. 8a & 8b show the internal circuit of the heater unit.

Fig. 9a & 9b show another preferred internal circuit of the heater unit.

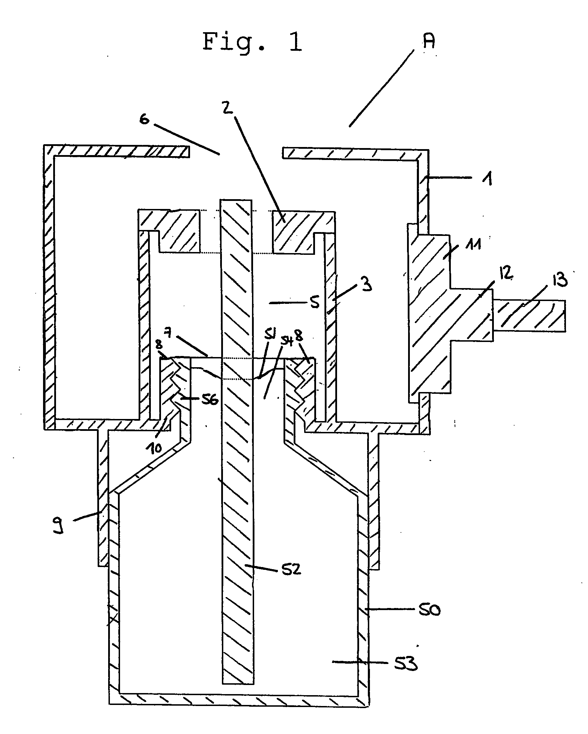

The thermal vaporizer A in Fig.1 has a housing 1, within the housing 1 a heater unit 2, which is preferably annular-shaped. The heater unit 2 is mounted on a support 3 which is fixed at the housing 1. The annular-shaped heater unit 2 and the support 3 form a passage 5 for a wick. The top of the housing 1 has an outlet 6 having the same axis as the passage 5 for the wick of the heater unit 2. The bottom the housing has a similar opening 7 having the same axis as the passage 5 of the wick of the heater unit 2. This opening 7 is surrounded by a mounting 8 which firmly holds a container 50. The container 50 is partly covered by a cover 9, which protects the container 50 from impact or damage to be applied or caused thereto from outside. Built into one side of the housing 1 is a circular portion 11 which can rotate about 90 degrees. Inserted into a central extension 12 of this circular portion 11 is an electrical contact 13.

The container 50 has a neck 54 with a neck insert 51 which tightly holds a form stable, porous wick 52, which is immersed in a liquid formulation 53, contained in the container 50. The upper part of the wick 52 is surrounded by the heater unit 2 whereas the lower part of the wick 52 reaches down to the bottom of the container 50.

The mounting 8 is formed on its inner periphery with a threaded portion 10 adapted for screw-thread engagement with a threaded portion 56 on the outer periphery of the neck 54 of the container 50. When the thermal vaporizer A is to be used, the container 50 is fixed into the mounting 8 by the screw-thread engagement of the threaded portions 10 and 56 whereby the wick 52 of the container 50 is inserted into the heater unit 2 concentrically therewith. Although the screw-thread engagement between the threaded portions 10 and 56 is resorted to for attaching the container 50 to the mounting 8 the threaded portions 10 and 56 may alternatively be replaced by a projection and an indentation which are engageable with each other.

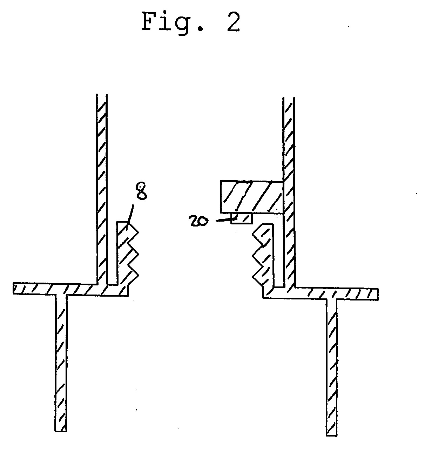

Fig.2 shows a preferred embodiment of the mounting 8 for the container 50 with an activating means 20 fixed in a sufficient distance above the mounting 8.

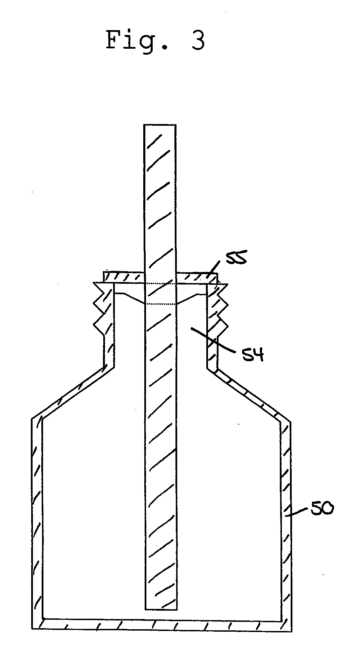

Fig. 3 shows a preferred embodiment of the means 55 of the container 50 for actuating the activating means 20 of the thermal vaporizer A, whereby said means 55 is a protruding part such as a molding on the neck 54.

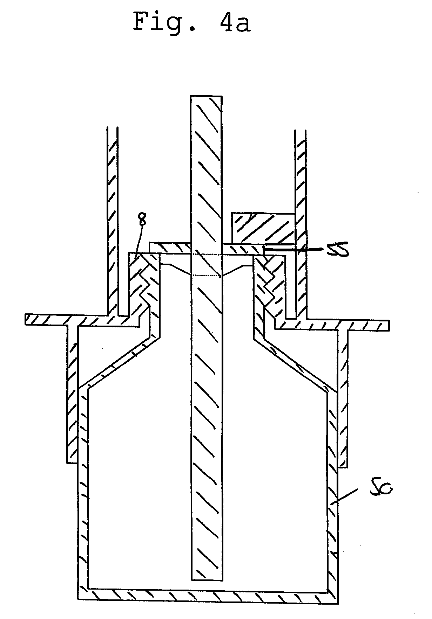

The container 50 shown in Fig. 4a comprises a means 55 in form of a protruding part, such as a molding, which actuate the activating means 20 of the thermal vaporizer A by pressing the activating means 20 with the means 55 when the container 50 is fixed into the mounting 8 of the thermal vaporizer A. The activating means 20 is actuated and thus not visible in Fig. 4a. After removing the container, the activating means 20 goes back into its original position. In a preferred embodiment this can be achieved by a spring fixed inside the activating means 20.

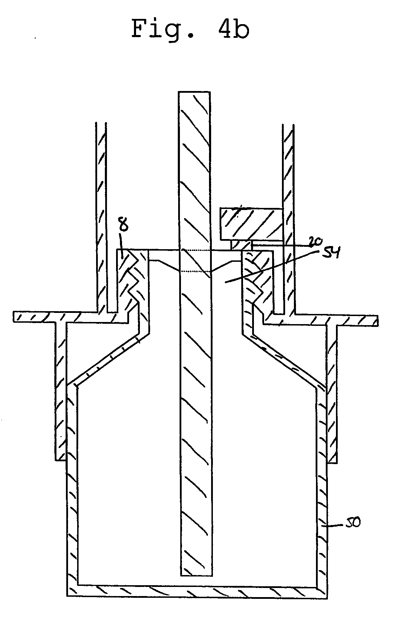

The sealing means of the container 50 shown in Fig. 4b finishes with the neck 54 of the container 50. Therefore, the activating means 20 is not actuated when the container 50 is fixed into the mounting 8 of the thermal vaporizer A.

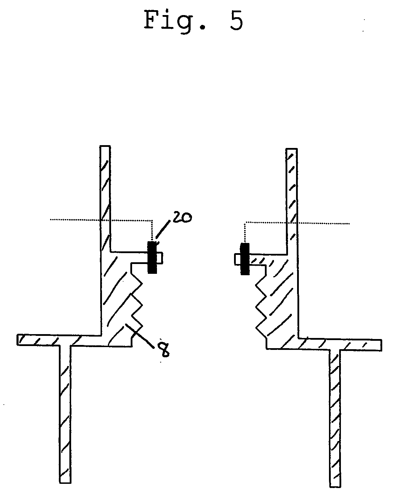

The activating means as shown in Fig. 5 are two electrical connections which are molded into the mounting 8 of the thermal vaporizer A.

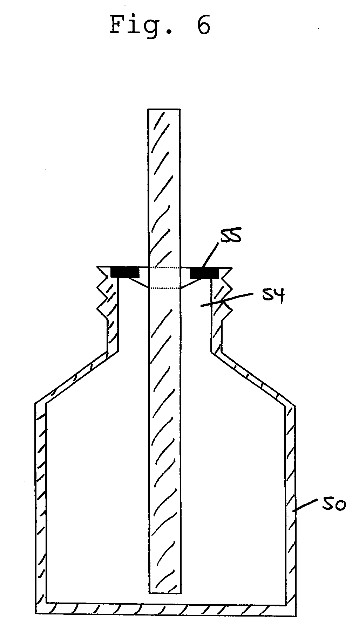

The means 55 shown in Fig 6 which actuate the activating means 20 are electrical contacts which are metal connectors on the neck 54 of the container 50.

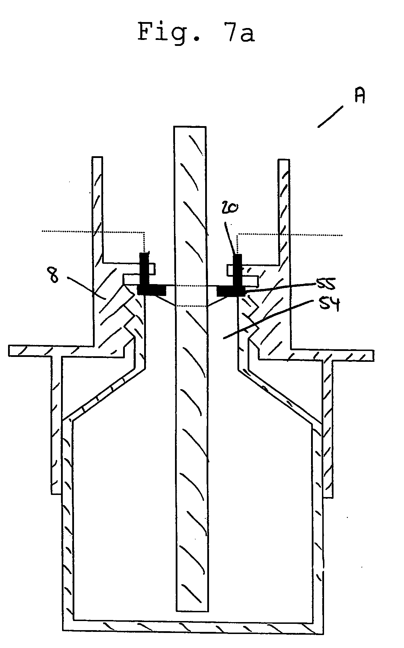

In Fig. 7a the container 50 which has the same embodiment as the one described in Fig 6 is fixed into the mounting 8 of the thermal vaporizer A. The means 55 on the neck 54 actuates the activating means 20 of the thermal vaporizer A which has the same embodiment as the one described in Fig. 5.

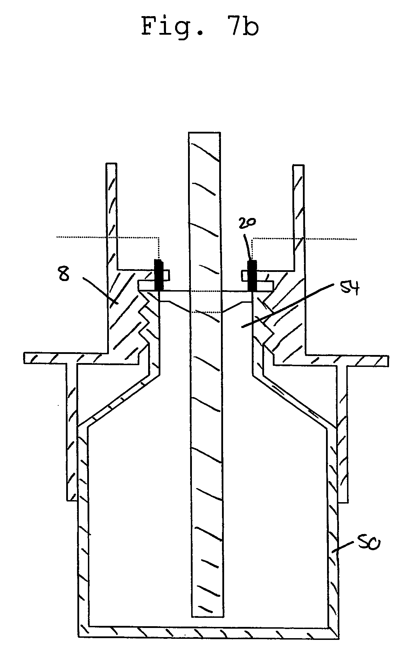

The sealing means of container 50 shown in Fig. 7b finishes with the neck 54 of the container 50. Therefore, the activating means 20 is not actuated when the container 50 is fixed into the mounting 8 of the thermal vaporizer A.

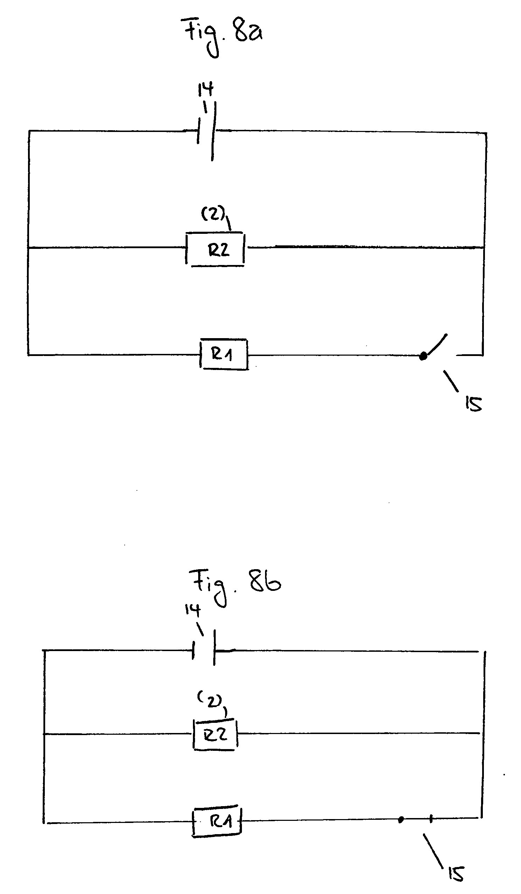

Fig. 8a & 8b show internal circuits of the heater unit 2.

In Fig. 8a in a primary circuit, the heater unit 2 (shown as resistor R2) is connected to the voltage source 14. A secondary circuit comprises a resistor R1 and a switch 15 which may be closed by the activating means 20. If the switch 15 is open, the resistor R1 has no effect on the heater unit 2.

Fig 8b shows the same circuit as described in Fig. 8a but in this case the switch 15 of the secondary circuit has been closed by the activating means 20, which has been actuated by the means 55 of the container 50, when said container 50 has been fixed into the mounting 8 of the thermal vaporizer A. In this case a part of the available power is taken up by the resistor R1 such that only a portion is available to the heater unit 2 which thus runs at a lower temperature.

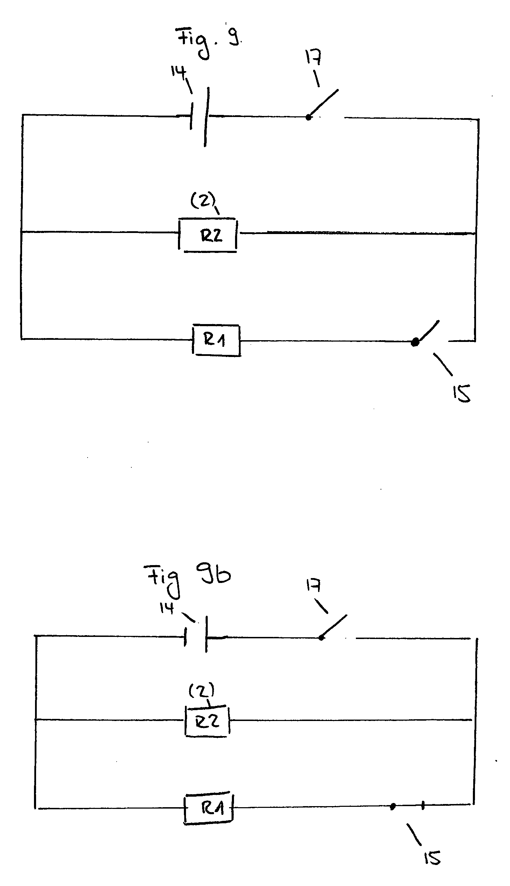

Fig. 9a & 9b show other preferred internal circuits of the heater unit 2.

Fig. 9a shows a similar internal circuit as described in Fig. 8 with an additional switch 17 which is closed when a container 50 is fixed into the mounting. Due to this switch the heater unit of thermal vaporizer A may only work, if a container is inserted.

Fig. 9b shows the same circuit as described in Fig. 9 with a closed secondary circuit.

## Claims

1. A thermal vaporizer (A) for a liquid formulation, comprising a volatile active, with a housing (1) having
a heater unit (2) for evaporating the liquid formulation,
a mounting (8) for a container (50) for the liquid formulation to be disposed under said heater unit (2),
a passage (5) for a wick (52) to be heated by the heater unit (2),
at least one outlet (6) for the evaporated volatile active,
an electrical contact connected to said heater unit (2),
**characterized by**
activating means (20) for two different predetermined heating temperatures of the heater unit (2)
said activating means (20) being activated by the container (50).

2. Thermal vaporizer (A) according to claim 1, wherein the activating means (20) is a switch.

3. Thermal vaporizer (A) according to claim 1, wherein the activating means (20) are interrupted circuits, at least one of which is closed a by metal connector on the container (50) when the container (50) is fixed into the mounting (8).

4. Thermal vaporizer (A) according to any of the preceding claims wherein the heater unit (2) has an annular shape enclosing the passage (5) of the wick (52).

5. Thermal vaporizer (A) according to any of the preceding claims, wherein the container (50) is fixed into the mounting (8) of the thermal vaporizer (A) by screw-thread engagement between an internally threaded portion of the mounting (8) of the thermal vaporizer (A) and an externally threaded portion of the container (50).

6. Thermal vaporizer (A) according to any of the preceding claims for a fragrance or a insecticide.

7. Container (50) for the thermal vaporizer (A) according to any of the preceding claims comprising means (55) which actuate the activating means (20) of the thermal vaporizer (A) when the container (50) is fixed into the mounting (8).

8. Container (50) according to claim 7, wherein the means (55) which actuate the activating means (20) is protruding part.

9. Container (50) according to claim 8, wherein the protruding part is a molding.

10. Container (50) according to claim 7, wherein the means (55) which actuate the activating means (20) is an metal connector.

11. Thermal vaporizer (A) comprising a container (50) according to claim 7.
